(19) Europäisches Patentamt
European Patent Office
Office européen des brevets

(11) Publication number: **0 044 536**
A1

(12) **EUROPEAN PATENT APPLICATION**

(21) Application number: 81105606.8

(22) Date of filing: 16.07.81

(51) Int. Cl.³: A 01 N 31/14
A 01 N 31/16, A 01 N 33/10
A 01 N 33/20, A 01 N 37/02
A 01 N 37/24, A 01 N 41/10

(30) Priority: 17.07.80 US 169599

(43) Date of publication of application:
27.01.82 Bulletin 82/4

(84) Designated Contracting States:
BE CH DE FR GB IT LI NL SE

(71) Applicant: J.T. Baker Chemical Co.
222 Red School Lane
Phillipsburg, New Jersey 08865(US)

(72) Inventor: Gray, Gary M.
1572 Ralston Road
Bethlehem Pennsylvania(US)

(74) Representative: Vossius.Vossius.Tauchner.Heunemann.Rauh
Siebertstrasse 4 P.O. Box 86 07 67
D-8000 München 86(DE)

(54) **Plant growth regulators and their use.**

(57) Benzyl phenyl ethers are disclosed as inhibitors of cytokinin plant growth regulatory activity and as possessing seed germination regulatory properties and senescence delaying activity when applied to plants. Benzyl phenyl ethers may also be useful as plant dwarfing agents, agents to retard seedling development or as herbicides.

EP 0 044 536 A1

Croydon Printing Company Ltd.

VOSSIUS · V... ...CHNER
HEUNEMA... ...
PATENTANWÄLTE
SIEBERTSTR. 4, ... ...CHEN 80
TEL. (089) 47 ...

15. Juli 1981
0044536



our Ref.:R 203 EP

## PLANT GROWTH REGULATORS AND THEIR USE

This invention relates to the use of benzyl phenyl ether compounds as plant growth regulating agents and to plant growth regulating compositions of said compounds.

### Summary of the Invention

According to the present invention it has been discovered that benzyl phenyl ethers of the general formula I

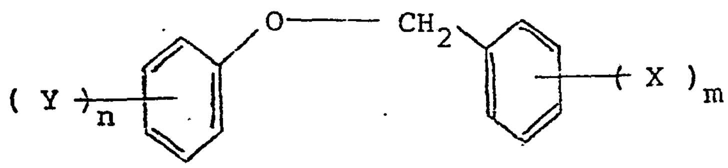

(I)

and phytopharmaceutically acceptable salts thereof, wherein X is selected from the group consisting of (lower)alkyl, halo, nitro, $-CF_3$, -S-(lower)alkyl, or -O-(lower)alkyl; Y is selected from the group consisting of (lower)alkyl, halo, nitro, $-CF_3$, -S-(lower)alkyl, -O-(lower)alkyl, -O-acetyl, hydroxy, -NH-acetyl, amino, -NH(lower)alkyl, -N-di(lower)-alkyl, $-SO_2$-(lower)alkyl, and -SO-(lower)alkyl; n is equal to 0, 1 or 2; and m is equal to 0, 1 or 2, have plant growth regulating activity. The term (lower)alkyl as used in the foregoing definitions is meant to include straight or branched chain alkyl radicals containing from 1 to 6 carbon atoms, such as methyl, butyl, hexyl or 1-methylethyl.

The phytopharmaceutically acceptable salts can be salts with inorganic or organic acids, such as hydrochloric, nitric, sulfuric, chloric, thiocyanic, phosphoric, acetic, p-toluenesulfonic, oxalic, methanesulfonic, ..

ethanesulfonic or naphthalenesulfonic acid.

The benzyl phenyl ethers and phytopharmaceutically acceptable salts thereof inhibit cytokinin plant growth regulating activity and possess seed germination regulating properties or senescence delaying activity when applied to plants in plant growth regulating amounts. Benzyl phenyl ethers and phytopharmaceutically acceptable salts thereof have also been found to possess plant dwarfing activity when applied to plants in plant dwarfing effective amounts and herbicidal activity when applied in herbicidally effective amounts.

The benzyl phenyl ether compounds of this invention have been found to possess plant growth regulating activity when employed in effective plant growth regulating amounts. The compounds of the present invention are generally active plant growth regulants when applications of from about 0.05 kg/hectare to about 20 kg/hectare of the compound is employed and preferably from about 0.1 kg to about 5 kg/hectare.

Especially preferred as plant growth regulating compounds of this invention are compounds of the general formula II

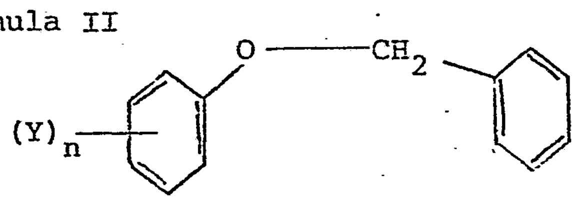

(II)

and phytopharmaceutically acceptable salts thereof wherein Y is selected from the group consisting of -S-(lower)alkyl, -O-acetyl, hydroxy, -NH-acetyl, amino and $-SO_2-$(lower)alkyl; n is equal to 1 and Y is in either the 3-, or 4- position of

the phenyl ring.

As examples of plant growth regulating compounds of this invention there may be mentioned

phenyl benzyl ether

3-(benzyloxy)phenol

4-(benzyloxy)phenol

1-(benzyloxy)-4-methylbenzene

1-(benzyloxy)-4-chlorobenzene

1-(benzyloxy)-4-nitrobenzene

1-(benzyloxy)-4-(trifluoromethyl)benzene

1-(benzyloxy)-4-(methylthio)benzene

1-(benzyloxy)-4-(ethoxy)benzene

O-acetyl-3-(benzyloxy)phenol

4-(benzyloxy)benzeneamine

4-(benzyloxy)benzeneamine hydrochloride

3-(benzyloxy)benzeneamine

3-(benzyloxy)benzeneamine hydrochloride

N-ethyl-4-(benzyloxy)benzeneamine

N,N-diethyl-4-(benzyloxy)benzeneamine

1-(benzyloxy)-4-(methylsulfonyl)benzene

1-(benzyloxy)-4-(methylsulfinyl)benzene

3-(4-chlorobenzyloxy)phenol

3-(4-methylbenzyloxy)phenol

(4-nitrobenzyloxy)benzene

(4-[trifluoromethyl]benzyloxy)benzene

3-(4-[methylthio]benzyloxy)phenol

3-(4-methoxybenzyloxy)phenol

N-acetyl-4-(benzyloxy)benzeneamine

N-acetyl-3-(benzyloxy)benzeneamine

3(2,4-dichlorobenzyloxy)phenol and

1-(benzyloxy)-2,4-dichlorophenol.

The compounds of this invention are generally known to be prepared according to procedures generally recognized in the art, for example, by the reaction of a phenol of the formula III

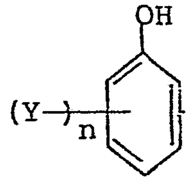

(III)

with a benzyl bromide or benzyl chloride of the formula IV

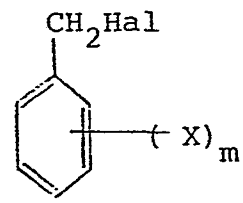

(IV)

in the presence of potassium carbonate at ambient temperature or by heating for from about 1 to 12 hours under a nitrogen atmosphere and in N,N-dimethylformamide wherein Y, X, m and n are as defined hereinbefore and Hal is chlorine or bromine. For example, the compounds can be prepared by mixing 10 mmols of the phenol, 10 mmols of the benzyl bromide and 20 mmols of potassium carbonate in 30 mmols N,N-dimethylformamide with stirring under nitrogen at atmospheric temperature for about 1 to about 12 hours before being poured into 300 ml of water. If the product is a solid, it is collected by vacuum filtration. If the product is an oil, it is extracted into 200 mls of diethyl ether and the solution dried with anhydrous magnesium sulfate before being filtered. The filtrate is evaporated to dryness to

yield the product.

As examples of preparations of compounds useful in this invention reference may be had to the following exemplary preparations.

Preparation 1

N-Acetyl-4-(benzyloxy)benzeneamine

In 20 ml of sieve dried dimethylformamide 1.27 g of 4-acetamidophenol, 1.44 g of benzyl bromide and 2.32 g of anhydrous potassium carbonate are combined and stirred at room temperature under nitrogen for about 16 hours. The reaction mixture is poured into distilled water and the mixture stirred for about an hour after which a white precipitate is collected by nitrogen pressure filtration. The precipitate is dried on the filter under a stream of nitrogen for two hours after first being washed with two 20 ml portions of distilled water. Approximately 1.91 g (94% yield) of white powder N-acetyl-4-(benzyloxy)benzeneamine is collected.

Preparation 2

4-(Benzyloxy)benzeneamine hydrochloride

The N-acetyl-4-(benzyloxy)benzeneamine of Preparation 1 is hydrolyzed by combining 1.79 g thereof with 1.34 g of potassium hydroxide (88%) and 20 ml of absolute ethanol and heating at reflux for about 16 hours. The reaction mixture is evaporated to dryness and washed with two 20 ml portions of distilled water. The residue is collected by vacuum filtration and dried on the filter. The hydrochloride salt of 4-(benzyloxy)benzeneamine is obtained by washing the residue with four 20 ml portions of diethylether and filtering. The filtrate is stirred as dry HCl is bubbled into the solution causing the precipitation of a white solid which is collected, washed with 20 mls of acetone and three 20 ml portions of diethyl ether and collected by nitrogen pressure filtration followed by drying on the filter under a nitrogen stream. Approximately 1.00 g of 4-(benzyloxy)-benzeneamine hydrochloride is obtained.

## Preparation 3

### O-Acetyl-3-(benzyloxy)phenol

In a manner similar to Preparation 1, 3.80 g of resorcinol acetate, 4.28 g benzyl bromide and 6.90 g of potassium carbonate in 50 ml dimethylformamide are mixed and stirred at room temperature overnight under nitrogen. Isolation and purification of the product produces 2.87 g of O-acetyl-3-(benzyloxy)phenol.

## Preparation 4

### 3-(Benzyloxy)phenol

In a procedure similar to Preparation 2, 2.42 g of O-acetyl-3-(benzyloxy)phenol is hydrolyzed with 10 ml of a 50% aqueous sodium hydroxide solution in 30 mls of methanol and yields 0.61 g of white crystalline 3-(benzyloxy)phenol.

## Preparation 5

### 1-(Benzyloxy)-4-(methylthio)benzene

In a procedure similar to Preparation 1, 1.40 g of 4-(methylmercapto) phenol, 1.71 g of benzyl bromide and 2.76 g of potassium carbonate are mixed in 30 ml of sieve dried

dimethylformamide and stirred for 16 hours under nitrogen and produces 2.29 g of solid white powder 1-(benzyloxy)-4-(methylthio)benzene.

Preparation 6

1-(Benzyloxy)-4-(methylsulfonyl)benzene

In 40 ml of methylene chloride there is dissolved with stirring 1.90 g 1-(benzyloxy)-4-(methylthio)benzene and 4.25 g 3-chloro-perbenzoic acid is added as the stirring continues. After being stirred for four hours, the reaction mixture is filtered and the filtrate evaporated to dryness. Recrystallization of the reaction product in methylene chloride and hexane yields 1.47 g of 1-(benzyloxy)-4-(methylsulfonyl)benzene.

Preparation 7

N-acetyl-3-(benzyloxy)benzeneamine

A mixture of 3.02 g of 3-acetamidophenol, 3.42 g of benzyl bromide and 5.52 g of anhydrous potassium carbonate in 40 ml of sieve dried dimethylformamide is stirred at room temperature under nitrogen for about 16 hours and the reaction mixture is poured into 300 ml of distilled water. The mixture is stirred for 15 minutes and then filtered. The resulting off-white, water-insoluble solid is taken up in 50 ml of hot methylene chloride and the solution heated under a nitrogen stream. When the volume of the solution reaches 40 ml, 20 ml of hexane is added after which the solution is treated with decolorizing carbon and filtered. The filtrate is again heated under a stream of nitrogen

until the volume of the solution reaches 50 ml at which point the solution is cooled to room temperature and placed in a freezer. A white crystalline solid forms and is collected by nitrogen pressure filtration. After being dried on the filter under a stream of nitrogen for one hour, 3.93 g N-acetyl-3-(benzyloxy)benzeneamine is obtained.

Preparation 8

3-(Benzyloxy)benzeneamine hydrochloride

The N-acetyl-3-(benzyloxy)benzeneamine of Preparation 7 is hydrolyzed by combining 2.41 g thereof with 1.30 g of potassium hydroxide (88%) and 18 ml of absolute ethanol and 4 ml water and heating at reflux for about 18 hours and allowed to cool to room temperature. After the ethanol is removed, the residue is treated with 50 ml water and 50 ml methylene chloride and the phases separated. The aqueous layer is then washed with an additional 50 ml of methylene chloride and the organic layers combined, dried with magnesium sulfate and filtered. The filtrate is evaporated to dryness and the oily residue taken up in 100 ml of diethyl ether. Dry HCl gas is bubbled into the solution for about 5 minutes and the insoluble precipitate is collected and dried by having air pulled through it overnight. Approximately 1.53 g (65% yield) of white powdered 3-(benzyloxy)benzeneamine hydrochloride is obtained.

The compounds of preparation nos. 1 to 8 were characterized by proton nmr. Results of the proton nmr characterization were as set forth in the following table

TABLE

| Compound of Preparation No.: | δ (ppm) | #H | Splitting Pattern |
|---|---|---|---|
| 1 | 7.33 | 9 | broad singlet |
| | 7.08 | | AB quartet |
| | 4.95 | 2 | singlet |
| | 2.01 | 3 | singlet |
| 2 | 7.32 | 9 | broad singlet |
| | 7.15 | | AB quartet |
| | 5.06 | 2 | singlet |
| 3 | 7.33 | 9 | broad singlet |
| | 7.0 | | complex multiplet |
| | 4.98 | 2 | singlet |
| | 2.23 | 3 | singlet |
| 4 | 7.33 | 9 | broad singlet |
| | 6.7 | | complex multiplet |
| | 5.33 | 1 | broad singlet |
| | 4.92 | 2 | singlet |
| 5 | 7.30 | 9 | broad singlet |
| | 7.10 | | AB quartet |
| | 4.95 | 2 | singlet |
| | 2.35 | 3 | singlet |

| | | | |
|---|---|---|---|
| 6 | 7.20 } 7.01 } | 9 | AB quartet<br>broad singlet |
| | 5.19 | 2 | singlet |
| | 3.12 | 3 | singlet |
| 7 | 8.0 | 1 | very broad singlet |
| | 7.33 } 6.9 } | 9 | broad singlet<br>complex multiplet |
| | 4.97 | 2 | singlet |
| | 2.07 | 3 | singlet |
| 8 | 7.1 | 9 | complex multiplet |
| | 5.07 | 2 | singlet |

The compounds of the invention have plant growth regulating activities. The plant growth regulating effects of the compounds are manifested as for example a stunting or dwarfing effect on the vegetative growth of plants. Such stunting or dwarfing may be useful, for example, in peanuts, cereals and soya bean where reduction in stem growth may reduce the risk of lodging and may also permit increased amounts of fertilizer to be applied. The stunting of woody species is useful in controlling the growth of undergrowth under power lines and the like. Compounds which induce stunting or dwarfing may also be useful in modifying the stem growth of sugar cane thereby increasing the concentration of sugar in the cane at harvest; in sugar cane, the flowering and ripening may be controllable by applying the compounds. Stunting of peanuts can assist in harvesting. Growth retardation of grasses can help maintenance of grass swards. The compounds may stunt grasses without significant

phytotoxic effects and without deleteriously affecting the appearance, particularly the color, of the grass. This makes such compounds attractive for use on ornamental lawns and on grass verges. They may also have an effect on flower head emergence in for example grasses. The compounds may also stunt weed species present in the grasses; examples of such weed species are sedges such as Cyperus spp. and dicotyledonous weeds, for example, daisy, plantain, knotweed, speedwell, thistle, docks and ragwort. The growth of non-crop vegetation, for example, weeds or cover vegetation, may be retarded thus assisting in the maintenance of plantation and field crops. In fruit orchards, particularly orchards subject to soil erosion, the presence of grass cover is important. However, excessive grass growth requires substantial maintenance. The compounds of the invention could be useful in this situation as they could restrict growth without killing the plants which would lead to soil erosion. At the same time, the degree of competition for nutrients and water by the grass would be reduced and this could result in an increased yield of fruit. In some cases, one grass species may be stunted more than another grass species; this selectivity could be useful for example for improving the quality of a sward by preferential suppression of the growth of undesirable species.

The dwarfing may also be useful in miniaturizing ornamental, household, garden and nursery plants, for example, poinsettias, chrysanthemums, carnations, tulips and daffodils.

As indicated above, the compounds may also be used to stunt woody species. This property may be used to control

hedgerows or to shape fruit trees, for example, apples.

The plant growth regulating effect may, as implied above, manifest itself in an increase in crop yield.

In the potato, vine control in the field and inhibition of sprouting in the store may be possible.

Other plant growth regulating effects caused by the compounds may include alteration of leaf angle and promotion of tillering in monocotyledonous plants. The former effect may be useful for example in altering the leaf orientation of, for example, potato crops thereby letting more light into the crops and inducing an increase in phytosynthesis and tuber weight. By increasing tillering in monocotyledonous crops, for example, rice, corn and soybeans, the number of flowering shoots per unit area may be increas-ed thereby increasing the overall yield of such crops. In grass swards an increase in tillering could lead to a denser sward which may result in increased resilience in wear.

The treatment of plants with the compounds may lead to the leaves developing a darker green color and delay senescence in crops thereby leading to improved pod fill in soybeans, for example.

The compounds may inhibit, or at least delay, the flowering of sugar beet and thereby may increase sugar yield. They may also reduce the size of sugar beet without reducing significantly the sugar yield thereby enabling an increase in planting density to be made. Similarly in other root crops, such as, for example, turnip, swede, mangold, parsnip, beetroot, yam and cassava, it may be possible to increase the planting density.

The compounds could be useful in restricting the vegetative growth of cotton thereby leading to an increase in cotton yield.

The compounds may be useful in rendering plants resistant to stress since the compounds can delay the emergence of plants grown from seed, shorten stem height and delay flowering. These properties could be useful in preventing frost damage in countries where there is significant snow cover in the winter since then the treated plants would remain below the snow cover during the cold weather. Further the compounds may provide drought or cold resistance in certain plants.

In carrying out the plant growth regulating method of the invention, the amount of compound to be applied to regulate the growth of plants will depend upon a number of factors, for example the particular compound selected for use, and the identity of the plant species whose growth is to be regulated. However, in general an application rate of 0.05 to 20, preferably 0.1 to 5, kg per hectare is used. However, on certain plants even application rates within these ranges may give undesired phytotoxic effects. Routine tests may be necessary to determine the best rate of application of a specific compound for any specific purpose for which it is suitable.

The compounds may be used as such plant growth regulating purposes but are more conveniently formulated into compositions for such usage. The invention thus provides also a plant growth regulating composition comprising a compound of Formula I or II or a phytopharmaceutically acceptable salt thereof as hereinbefore defined, and an inert carrier or diluent.

It also provides a method of regulating the growth of a plant which method comprises applying to the plant, to seed of the plant or to the locus of the plant or seed a plant growth regulating compound as hereinbefore defined.

The plant growth regulating compounds of this invention can be applied in a number of ways, for example they can be formulated or unformulated, directly to the foliage of a plant, or they can be applied also to bushes and trees, to seeds or to other medium in which plants, bushes or trees are growing or are to be planted, or they can be sprayed on, dusted on or applied as a cream or paste formulation, or they can be applied as a vapor. Application can be to any part of the plant, bush or tree, for example to the foliage, stems, branches or roots, or to soil surrounding the roots, for systemic activity, or to the seed before it is planted.

The term "plant" as used herein includes seedlings, bushes and trees.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum Hewitt's earth, diatomaceous earth and China clay. Such granules can be

preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent, for example a mineral oil, for assisting the adhesion of the composition to the seed. Alternatively the active ingredient can be formulated for seed dressing purposes using an organic solvent, for example n-methylpyrrolidone or dimethylformamide.

The compositions may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient in an organic solvent optionally containing wetting, dispersing or emulsifying agents and then adding the mixture to water which may also contain wetting, dispersing or emulsifying agents. As example of suitable organic solvents there may be mentioned, for example, ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers such as 2-ethoxy-ethanol and 2-butoxyethanol.

The compositions to be used as sprays may also be in the form of aerosols wherein the formulation is held in a container under pressure in the presence of a propellant,

such as, for example, fluorotrichloromethane or dichlorodifluoromethane.

The plant growth regulating compounds can be mixed in the dry state with a pyrotechnic mixture to form a composition suitable for generating in enclosed spaces a smoke containing the compounds. Alternatively, the compounds may be used in a micro-encapsulated form.

By including suitable additives, for example additives for improving the distribution, adhesive power and resistance to rain on treated surfaces, the different compositions can be better adapted for various utilities.

The plant growth regulating compounds can also be used as mixtures with fertilizers. Compositions comprising only granules of fertilizer incorporating, for example coated with, the plant growth regulating compound, are preferred. Such granules suitably contain up to 25% by weight of the compound. The invention therefore also provides a fertilizer composition comprising the plant growth regulation compound of Formula I.

The compositions may also be in the form of liquid preparations for use as dips or sprays which are generally aqueous dispersions or emulsions containing the active ingredient in the presence of one or more surfactants, for example, wetting agents, dispersing agents, emulsifying agents or suspending agents. These agents can be cationic, anionic or non-ionic agents. Suitable cationic agents

are quaternary ammonium compounds, for example cetyltrimethylammonium bromide.

Suitable anionic agents are soaps, salts of aliphatic monoesters of sulfuric acid, for example sodium lauryl sulfate, and salts of sulfonated aromatic compounds, for example sodium dodecylbenzenesulfonate, sodium, calcium or ammonium lignosulfonate, butylnaphthalene sulfonate, and a mixture of sodium diisopropyl- and triisopropyl-naphthalene sulfonates.

Suitable non-ionic agents are the condensation products of ethylene oxide with fatty alcohols such as oleyl or cetyl alcohol, or with alkyl phenols such as octyl- or nonylphenol and octylcresol. Other non-ionic agents are the partial esters derived from long chain fatty acids and hexitol anhydrides, the condensation products of the acid partial esters with ethylene oxide, and the lecithins. Suitable suspending agents are hydrophilic colloids, for example polyvinylpyrrolidone and sodium carboxymethylcellulose, and the vegetable gums, for example gum acacia and gum tragacanth.

The compositions for use as aqueous dispersions or emulsions are generally supplied in the form of a concentrate containing a high proportion of the active ingredient. The concentrates are then diluted with water before use. These concentrates often should be able to withstand storage for prolonged periods and after such storage be capable of dilution with water in order to form aqueous preparations which remain homogeneous for a sufficient time to enable them to be applied by conventional spray equipment. The concentrates may conveniently contain up to 95%, suitably 10-85%, for example 25-60%, by weight of the active ingredient. These concentrates suitably contain organic acids, for example, alkaryl or aryl sulfonic acids such as xylenesulfonic acid or dodecylbenzenesulfonic acid, since the presence of such acids can increase the solubility of the active ingredient in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient depending upon the intended purpose, but an aqueous preparation containing 0.0005% or 0.01% to 10% by weight of active ingredient may be used.

The compositions of this invention can comprise also other compounds having biological activity, for example, compounds having similar or complementary plant growth regulating activity or compounds having herbicidal or insecticidal or fungicidal activity.

The fungicidal compound can be for example one which is capable of combatting ear diseases of cereals such as wheat, seed and soil borne diseases and downy and powdery mildews on grapes and powdery mildew and scab on apple and the like. Examples of the other fungicidal compounds are imazalil, benomyl, carbendazim (BCM), thiophanate-methyl, captafol, captan, sulphur, dithiocarbamates, carbathiins, copper oxychloride, triforine, dodemorph, tridemorph, dithianon, pyrazophos, binapacryl, quinomethionate, panoctine, furalaxyl, aluminum trio(ethyl phosphonate), DPX3217, ethirimol, dimethirimol, bupirimate, chlorothalonil, Chevron RE 20615,

vinclozolin, procymidone, iprodione and metaxanine.

The other plant growth regulating compound can be one which controls weeds or seedhead formation, improves the level or longevity of the plant growth regulating activity of the compounds of this invention, selectively controls the growth of the less desirable plants such as grasses or causes the plant growth regulating compound of Formula 1 to act faster or slower as a plant growth regulating agent. Some of these other agents will be herbicides. Examples of suitable agents are the gibberellins such as $GA_3$, $GA_4$, or $GA_7$, the auxins such as indoleacetic acid, indolebutyric acid, naphthoxyacetic acid, naphthylacetic acid, phenoxyacetic acids such as 2,4-D or MCPA, substituted benzoic acids such as TIBA and morphactins such as chlorfluorecol and the cytokinins such as kinetin, benzylademine, BAP, diphenylurea and benzimidazole. Also included would be maleic hydrazide, glyphosate, glyphosine, long chain fatty alcohols and acids, dikegulac, substituted quaternary ammonium and phosphonium compounds such as CCC or Phosfon-D, carbetamide, asulam, abscissic acid, isopyrimol, and hydroxybenzonitriles such as bromoxynil.

Utilizing the compounds of this invention as the active ingredients in plant growth regulating compositions, said compounds were found to possess plant growth regulating activity when tested in accordance with the following test procedures.

<u>TEST A - Soybean Cotyledon Callus Growth Bioassay</u>

Cytokinin type activity is illustrated by a soybean

cotyledon callus bioassay test comprising the following procedure. Growth medium employed in this test is obtained in the following manner. One liter of growth medium is prepared by adding 10 ml of each of the sixteen following stock solutions to a two liter flask containing 250 ml of water:

| Stock Solution No. | Description |
|---|---|
| 1 | 30 grams $KH_2PO_4$ in 500 ml water |
| 2 | 100 grams $KNO_3$ in 500 ml water |
| 3 | 100 grams $NH_4NO_3$ in 500 ml water |
| 4 | 50 grams $Ca(NO_3)_2 \cdot 4H_2O$ in 500 ml water |
| 5 | 7.15 grams $MgSO_4 \cdot 7H_2O$ in 500 ml water |
| 6 | 6.5 grams KCl in 500 ml water |
| 7 | 1.4 grams $MnSO_4 \cdot 4H_2O$ in 500 ml water |
| 8 | Dissolve 1340 mg EDTA $Na_2 \cdot 2H_2O$ in 500 ml water and heat; while still hot add 990 mg $Fe_2SO_4 \cdot 7H_2O$ and stir into solution |
| 9 | 380 mg $ZnSO_4 \cdot 7H_2O$ in 500 ml water |
| 10 | 160 mg $H_3BO_3$ in 500 ml water |
| 11 | 75 mg KI in 500 ml water |
| 12 | 35 mg $Cu(NO_3)_2 \cdot 3H_2O$ in 500 ml water |
| 13 | 10 mg $(NH_4)_6MO_7O_{24} \cdot 4H_2O$ in 500 ml water |
| 14 | 10 grams i-inositol in 50 mg water |
| 15 | 50 mg nicotinic acid, 20 mg pyridoxine·HCl and 20 mg thiamin·HCl in 500 ml water |
| 16 | 40 mg α-naphthaleneacetic acid in 100 ml water. |

To said two liter flask is added 30 grams sucrose and the volume is adjusted to 450 ml and to pH 5.8 and thereafter

the volume is adjusted to 500 ml with deionized water.

Into each of seven 500 ml flasks (numbered 1 through 7) there is placed 125 ml of sterilized growth medium. Flask number 1 is adjusted to 250 ml volume by the addition of deionized water and serves as the growth medium control. Into each of flasks number 2 and 3 there is added 6.5 ml of 22 mg/l kinetin stock solution and each of the flasks volume is adjusted to 250 ml by the addition of deionized water. Flask 2 serves as the kinetin reference standard. Deionized water is added to each of flasks 4 through 7 to adjust the volume of each to 250 ml. This is followed by the addition and solubilization of 2.5 g Difco Bacto-Agar in each of the seven flasks. All seven flasks and their contents are then sterilized in an autoclave at 121°C and 15 psi for 15 minutes. Filtered (Millipore) and sterilized solutions of test compound are added to flasks 3 through 7 in amounts to result in a final concentration of test compound in the flasks in the following amounts:

| | |
|---|---|
| Flask 3 | 50 mg/l test compound (plus kinetin) |
| Flask 4 | .05 mg/l test compound |
| Flask 5 | .5 mg/l test compound |
| Flask 6 | 5 mg/l test compound |
| Flask 7 | 50 mg/l test compound. |

From each of the seven 500 ml flasks 50 ml of solution is withdrawn and deposited asceptically into sterile 125 ml flasks and plugged with cotton. To each 125 ml flask is added three small, approximately 5 mg fresh weight, soybean cotyledon callus pieces. The flasks are placed in a growth chamber at 27-30°C for twenty seven days after which the

callus is removed and observed for size and weight.

TEST B - Chlorophyll Retention Test

Six petri dishes are prepared by placing the following solutions into the respective dishes.

| Petri Dish No. | Solution |
|---|---|
| 1 | 25 ml distilled water |
| 2 | 25 ml aqueous solution of 5 mg/l kinetin |
| 3 | 25 ml aqueous solution of 5 mg/l kinetin and 50 mg/l test compound |
| 4 | 25 ml aqueous solution of 0.5 mg/l test compound |
| 5 | 25 ml aqueous solution of 5 mg/l test compound |
| 6 | 25 ml aqueous solution of 50 mg/l test compound |

Approximately 1 g of previously cut leaves (for example, 7 to 9 day old, wheat, oat or barley, but preferably oat) is floated on the solution in each petri dish. The petri dishes are stored under light of about 25 ft-candles at 27-30°C for approximately seven days. The leaves from each petri dish are then analyzed for the amount of chlorophyll retained in the leaves according to the following procedure. The chlorophyll is extracted from the leaves with several washes of 80% acetone at a total volume equivalent to 1 ml acetone for every 10 mg fresh weight of plant leaves after the last extraction. When the leaves appear to be free of green pigment, the total volume is adjusted with 100% acetone to accomodate for evaporative loss during extraction.

Optical density readings on a spectrophotometer are taken of the extract against a solvent blank (80% acetone) at wavelengths of 645 and 663 nm. The amount of chlorophyll is calculated with the following equation:

$$\text{Mg total chlorophyll/g tissue} = [20.2(D_{645}) + 8.02(D_{663})] \times \frac{V}{1000 \times W}$$

wherein D=optical density or absorbance

V=final volume of 80% acetone/chlorophyll extract

W=fresh weight in gram of the tissue extracted.

TEST C - Seed Germination Test

In each of 10 petri plates there is placed a disc of Whatman No. 1 filter paper. Into each petri plate containing the filter paper disk there is pipetted 5 ml water with or without kinetin or test compound as set forth in the Table hereinafter. Approximately 50 seeds are evenly sprinkled on each petri plate. Plates 5 through 10 are placed in a steel cannister to exclude light. The seeds on all the plates are allowed to germinate for 48 hours in an incubator set at 27-30$^{o}$C. The test is terminated by counting the number of seeds germinated and non-germinated and calculating the percent germination for all treatments. Any root elongation phenomenon is observed and noted. The solutions added to the ten petri plates and the treatment to which each is subjected is as follows.

| Petri Plate No. | Solution Added | Treatment |
|---|---|---|
| 1 | water | light |
| 2 | kinetin (10 mg/l) | light |
| 3 | kinetin (10 mg/l) + test compound (50 mg/l) | light |

| | | |
|---|---|---|
| 4 | test compound (50mg/l) | light |
| 5 | water | darkness |
| 6 | kinetin (10mg/l) | darkness |
| 7 | kinetin (10 mg/l) + test compound (50mg/l) | darkness |
| 8 | test compound (0.5 mg/l) | darkness |
| 9 | test compound (5 mg/l) | darkness |
| 10 | test compound (50 mg/l) | darkness |

The following table summarizes the results and observations made in accordance with the hereinbefore set forth Tests A, B and C.

| Compound of Preparation No. | Test | Concentration of Test Compound | Observation |
|---|---|---|---|
| 1 | B | 50 mg/l | kinetin inhibitor-reduced chlorophyll retention |
| 2 | A | 50 mg/l | inhibits action of kinetin-reduced callus growth |
| | B | 50 mg/l | counteracts action of kinetin-decreases chlorophyll retention |
| | C | .5&5mg/l | increased lettuce seed germination in darkness |
| | | 50 mg/l | decreased lettuce seed germination in darkness |
| | | 50 mg/l | counteracts action of kinetin in both light and darkness |
| 3 | B | 5&50 mg/l | increases chlorophyll retention |
| 4 | A | 50 mg/l | blocks action of kinetin-no growth of callus |
| | B | 5 mg/l | increased chlorophyll retention |
| 5 | B | 50 mg/l | counteracts action of kinetin-decreases chlorophyll retention |
| 6 | A | 50 mg/l | inhibits action of kinetin-reduces callus growth |

| | | | |
|---|---|---|---|
| | B | 50 mg/l | inhibits action of kinetin-reduces chlorophyll retention |
| 7 | B | 50 mg/l | increases chlorophyll retention |
| 8 | B | 5 mg/l | increases chlorophyll retention |
| | | 50 mg/l | inhibits action of kinetin-reduces chlorophyll retention. |

Although this invention has been described with respect to specific modifications, the details thereof are not to be construed as limitations, for it will be apparent that various equivalents, changes and modifications may be resorted to without departing from the spirit and scope thereof and it is understood that such equivalent embodiments are intended to be included herein.

CLAIMS

1. A plant growth regulating composition comprising an inert carrier material and from 5 to 95 parts by weight of a benzylphenylether of the formula I

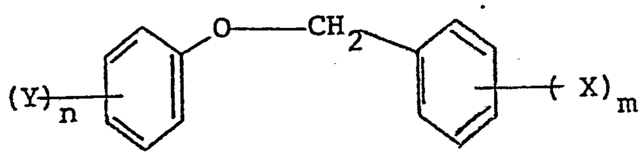

(I)

and phytopharmaceutically acceptable salts thereof, wherein X is selected from the group consisting of (lower)alkyl, halo, nitro, $-CF_3$, -S-(lower)alkyl, -O-(lower)alkyl; Y is selected from the group consisting of (lower) alkyl, halo, nitro, $-CF_3$, -S-(lower)alkyl, -O-(lower)alkyl, -O-acetyl, hydroxy, -NH-acetyl, amino, -NH-(lower) alkyl, N-di(lower)alkyl and $-SO_2$-(lower)alkyl, and -SO-(lower)alkyl); n is equal to 0, 1 or 2 and m is equal to 0, 1 or 2.

2. A plant growth regulating composition of claim 1 wherein the plant growth regulating compound is one wherein m is equal to zero, n is equal to 1 and Y is selected from the group consisting of -S-(lower)alkyl, -O-acetyl, hydroxy, -NH-acetyl, amino and $-SO_2$-(lower)alkyl and Y is on either the 3- or 4- position of the phenyl ring.

3. A plant growth regulating composition of claim 1 wherein the plant growth regulating compound is one wherein the compound is selected from the group consisting of 3-(benzyloxy)phenol, 1-(benzyloxy)-4-(methylthio)benzene, O-acetyl-3-(benzyloxy)phenol, 4-(benzyloxy)benzeneamine hydrochloride, 1-(benzyloxy)-4-(methylsulfonyl)benzene, N-acetyl-4-(benzyloxy)benzeneamine, N-acetyl-3-(benzyloxy)benzenamine and 3-(benzyloxy)benzeneamine hydrochloride.

4. A plant growth regulating composition of claim 1 wherein the plant growth regulating compound is 4-(benzyloxy)-benzeneamine hydrochloride.

5. Use of the benzylphenylether as set forth in any of the claims 1 to 4 for regulating the growth of leguminous plants.

6. Use according to claim 6 wherein the benzylphenylether is applied to the plant locus in an amount of from 0.05 to 20 kg/hectare.

0044536

European Patent Office

# EUROPEAN SEARCH REPORT

Application number

EP 81 10 5606

## DOCUMENTS CONSIDERED TO BE RELEVANT

| Category | Citation of document with indication, where appropriate, of relevant passages | Relevant to claim |
|---|---|---|
| A | FR - A - 2 204 351 (NIPPON SODA)<br>& DE - A - 2 252 818<br>& NL - A - 72 14611<br>---- | |

CLASSIFICATION OF THE APPLICATION (Int. Cl.3)

A 01 N 31/14
31/16
33/10
33/20
37/02
37/24
41/10

TECHNICAL FIELDS SEARCHED (Int. Cl.3)

A 01 N 31/16
33/10
37/02
37/24
41/10

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 22-10-1981 | DECORTE |

EPO Form 1503.1 06.78